# FASCICULE DE BREVET EUROPEEN

(11) **EP 0 613 361 B1**
(45) Date de publication et mention de la délivrance du brevet: **29.01.1997**
(21) Numéro de dépôt: 93901788.5
(22) Date de dépôt: 20.11.1992
(51) Int. Cl.: A61F 13/15

(54) **ARTICLE D'HYGIENE ABSORBANT JETABLE TEL QUE COUCHE-CULOTTE, A EFFET DE BARRIERE LATERALE AMELIORE**
ABSORBIERENDER HYGIENISCHER WEGWERFARTIKEL, WIE ETWA EINE WINDEL, MIT VERBESSERTEM SEITENSCHUTZ
DISPOSABLE ABSORBENT SANITARY ARTICLE SUCH AS A NAPPY WITH IMPROVED LATERAL BARRIER EFFECT

(30) Priorité: 22.11.1991 FR 9114399
(43) Date de publication de la demande: 07.09.1994
(73) Titulaire: PEAUDOUCE, 59126 Linselles (FR)
(72) Inventeur: VANDEMOORTELE, Philippe, F-59000 Lille (FR); KOCZAB, Jean-Pierre, F-59910 Bondues (FR); VILLEZ, Albert, F-59126 Linselles (FR)
(74) Mandataire: Casalonga, Axel
(86) Numéro de dépôt international: FR9201076
(87) Numéro de publication internationale: WO9309739

(56) Documents cités:
- EP-A- 0 243 013
- EP-A- 0 391 476
- US-A- 4 808 178

## Description

La présente invention se rapporte à un article d'hygiène absorbant jetable, notamment une couche-culotte, du type comprenant une feuille de support extérieure imperméable aux liquides, une feuille de couverture intérieure perméable aux liquides, un coussin absorbant disposé entre lesdites feuilles, des éléments élastiques longitudinaux fixés à l'état tendu à ladite feuille de support, à l'extérieur des deux bords longitudinaux du coussin absorbant, deux volets latéraux élastifiés fixés à la feuille de couverture le long des bords longitudinaux de l'article d'hygiène, et des moyens d'attache pour fermer l'article d'hygiène autour de la taille d'un utilisateur.

Des articles d'hygiène de ce type sont connus par la demande de brevet GB-A-2 161059 et par d'autres documents plus récents, par exemple les demandes de brevet EP-A-219 326, 243 013, 374 640 et 391 476.

Un article d'hygiène de ce type présentant une forme générale rectangulaire peut comporter deux découpes latérales opposées conférant à l'article d'hygiène une forme dite anatomique ou en sablier. Un tel article d'hygiène, qu'il soit pourvu ou non de telles découpes latérales, présente dans le sens de sa longueur, une partie arrière correspondante à une zone d'extrémité longitudinale, une partie avant correspondant à l'autre zone d'extrémité longitudinale une partie d'entrejambes correspondant à la zone intermédiaire de la couche de la couche-culotte. Sur un article d'hygiène de forme anatomique ou en sablier, la partie d'entrejambes présente une largeur plus faible que la partie avant et la partie arrière.

Les volets latéraux dont sont pourvus les articles d'hygiène suivant les documents antérieurs précités ont pour but d'améliorer l'étanchéité en générale de l'article d'hygiène dans la partie d'entrejambes, ainsi que l'effet de confinement de l'urine et des matières fécales. Ces volets latéraux formant effet de barrière latérale sont disposés sur la face intérieure de la feuille de couverture, c'est-à-dire la face en contact avec la peau de l'utilisateur, et sont espacés transversalement de manière à s'étendre sensiblement le long des bords longitudinaux de l'article d'hygiène. Ces volets latéraux peuvent, soit être formés directement dans le matériau de la feuille de couverture, comme proposé par la demande de brevet GB-A-2 161 059, soit être constitués par des rubans en matériau perméable ou imperméable aux liquides, fixés sur la face intérieure de la feuille de couverture. Dans tous les cas, ces volets sont reliés par l'un de leurs bords longitudinaux, dit bord proximal, à la feuille de couverture et portent, à leur autre bord longitudinal dit bord distal, c'est-à-dire leur bord libre, un élément élastique composé par exemple d'un ou de plusieurs fils ou brins élastiques, fixés de préférence par collage à l'état tendu au volet. Cet élément élastique peut par exemple être disposé dans une gaine ou un gousset formé par le repli sur lui-même du bord longitudinal du ruban, au moins sur la partie médiane de la longueur de ce ruban correspondant à la zone d'entrejambes de l'article d'hygiène, l'élément élastique pouvant être fixé au volet soit sur toute sa longueur, soit uniquement à ses deux extrémités. Dans les deux zones d'extrémités longitudinales de l'article d'hygiène, les bords distaux des volets sont fixés à la feuille de couverture, par exemple par une ligne ou bande de collage transversale, de telle manière que les volets se trouvent ici disposés à plat sur la feuille de couverture, les bords distaux des deux volets opposés étant orientés, soit vers l'intérieur, c'est-à-dire l'un vers l'autre, soit vers l'extérieur, c'est-à-dire éloignés l'un de l'autre.

La première solution pose des problèmes lorsque les bords proximaux des volets, dans le cas d'un article d'hygiène de forme anatomique ou en sablier, sont disposés au-dessus du coussin absorbant dans la partie d'entrejambes de largeur réduite, dans la mesure où les bords distaux des deux volets opposés sont alors trop proches l'un de l'autre, ce qui limite l'étanchéité et l'effet de confinement de l'urine et des matières fécales dans le cas d'articles d'hygiène dont le coussin absorbant présente également une largeur réduite dans cette partie d'entrejambes.

Les solutions proposées par les autres documents antérieurs précités consistent généralement à fixer les volets de telle manière que leurs bords proximaux se trouvent, dans la partie d'entrejambes de l'article d'hygiène, situés à l'extérieur des bords latéraux du coussin absorbant, c'est-à-dire dans une zone où la feuille de couverture est fixée à la feuille de support. Dans ce cas, en raison de l'épaisseur du coussin absorbant, ces volets doivent présenter une hauteur relativement importante pour pouvoir procurer un effet de barrière satisfaisant et une grande indépendance par rapport à la partie restante de l'article d'hygiène (coussin absorbant, feuille de support extérieure, éléments élastiques longitudinaux fixés à la feuille de support extérieur), ce qui augmente le coût de l'article de l'article d'hygiène équipé de tels volets latéraux.

Il apparaît donc que les solutions proposées jusqu'à présent ne donnent pas entière satisfaction.

La présente vise un article d'hygiène du type défini ci-dessus, notamment un article d'hygiène de forme dite anatomique ou en sablier, avec un coussin absorbant de largeur réduite dans la partie d'entrejambes, sur lequel les volets latéraux sont conçus de manière à procurer un effet de barrière optimal à un coût réduit,

L'article d'hygiène absorbant jetable conforme à l'invention, notamment une couche-culotte, présente une forme générale sensiblement rectangulaire avec des bords longitudinaux opposés et des bords transversaux opposés et comprend, de l'extérieur vers l'intérieur, une feuille de support imperméable aux liquides, un coussin absorbant disposé et fixé sur la face intérieure de la feuille de support, les dimensions du coussin étant inférieures à celle de la feuille de support, et une feuille de couverture perméable aux liquides recouvrant au moins partiellement la face intérieure du coussin absorbant et fixée à la feuille de support au moins partiellement, sur le pourtour du coussin absorbant. Cet article comprend, en outre, des éléments élastiques longitudinaux fixés à l'état tendu à la feuille de support, à l'extérieur des bords longitudinaux du coussin absorbant. L'article comprend, par ailleurs, deux volets latéraux espacés transversalement, disposés sur la face intérieure de la feuille de couverture, sensiblement le long des bords longitudinaux de l'article d'hygiène, lesdits volets présentant chacun un bord proximal relié à la feuille de couverture et un bord distal comportant un élément élastique longitudinal tendu. De plus, l'article d'hygiène comprend des moyens d'attache, au voisinage de l'un de ses bords transversaux, pour fermer l'article d'hygiène autour de la taille d'un utilisateur de telle manière que l'article d'hygiène définisse une partie avant et une partie arrière correspondant respectivement aux deux zones d'extrémité proches desdits bords transversaux opposés de l'article d'hygiène, et une partie d'entrejambes correspondant à la zone intermédiaire située entre lesdites deux zones d'extrémité. Suivant l'invention, chacun desdits volets latéraux est fixé à la feuille de couverture le long de son bord proximal, sur toute la longueur de l'article d'hygiène, de manière que ledit bord proximal se trouve au-dessus du coussin absorbant sur toute la longueur de l'article d'hygiène, y compris dans la partie d'entrejambes. Ce volet est replié sur lui-même, sur toute sa longueur, et est fixé à la feuille de couverture dans les parties d'extrémités de l'article d'hygiène de telle manière que son bord distal élastifié se trouve sensiblement au-dessus de son bord proximal, sur toute la longueur du volet.

Ainsi, le bord proximal de chaque volet latéral, du fait de sa disposition au-dessus du coussin absorbant, se trouve situé au plus près de la peau de l'utilisateur. De plus, du fait que le bord distal élastifié de chaque volet se trouve situé sensiblement au-dessus du bord proximal, les bords distaux des deux volets sont suffisamment éloignés l'un de l'autre et peuvent suivre de façon optimale les mouvements de l'utilisateur, conservant ainsi aux volets toujours leur effet de barrière. Par ailleurs, le bord proximal des volets se trouve éloigné au maximum des éléments élastiques longitudinaux fixés à la feuille de support extérieure, ce qui donne au volet une grande indépendance par rapport aux autres parties de la couche-culotte, notamment le coussin absorbant et les éléments élastiques longitudinaux fixés à la feuille de support.

Dans le cadre de l'invention, chaque volet peut être replié sur lui-même vers l'extérieur ou, de préférence, vers l'intérieur. Dans ce dernier cas, chaque volet forme de lui-même, dans la partie d'entrejambes, une poche lors du soulèvement de son bord distal élastifié par rapport à son bord proximal, c'est-à-dire par rapport à la feuille de couverture, améliorant ainsi l'effet de barrière.

En se référant aux dessins schématiques annexés, on va décrire ci-après plus en détail plusieurs modes de réalisation illustratifs et non limitatifs d'un article d'hygiène conforme à l'invention ; sur les dessins :
la figure 1 est une vue sur la face intérieure d'une couche-culotte conforme à l'invention disposée à plat ;
la figure 2 est une coupe partielle, à plus grande échelle, suivant II-II de la figure 1, dans une partie d'extrémité de la couche-culotte ;
la figure 3 est une coupe partielle, à plus grande échelle, suivant III-III de la figure 1, dans la partie d'entrejambes de la couche-culotte;
les figures 4 et 5 sont des coupes partielles, correspondant aux figures 2 et 3, d'une variante de réalisation d'une couche-culotte conforme à l'invention ;
les figures 6 et 7 sont des coupes partielles, correspondant aux figures 2 et 3, d'une autre variante de réalisation d'une couche-culotte conforme à l'invention ;
les figures 8 et 9 sont des coupes partielles, correspondant aux figures 2 et 3, d'encore une autre variante de réalisation d'une couche-culotte conforme à l'invention.

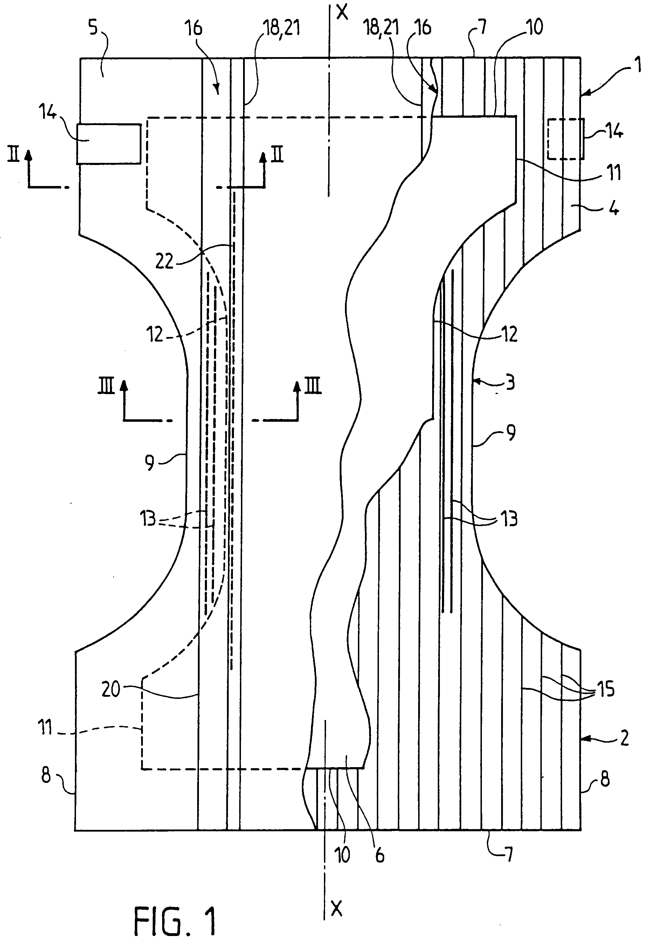

La couche -culotte à jeter telle qu'illustrée par les figures 1 à 3 est une couche-culotte dite anatomique ou en sablier, ayant deux découpes latérales opposées permettant de définir, dans le sens de la longueur de la couche-culotte, une partie arrière 1 correspondant à une zone d'extrémité longitudinale, une partie avant 2 correspondant à l'autre zone d'extrémité longitudinale et une partie d'entrejambes 3 correspondant à la zone intermédiaire de la couche-culotte. Dans la partie d'entrejambes 3, la couche-culotte présente une largeur plus faible que dans la partie avant 1 et dans la partie arrière 2.

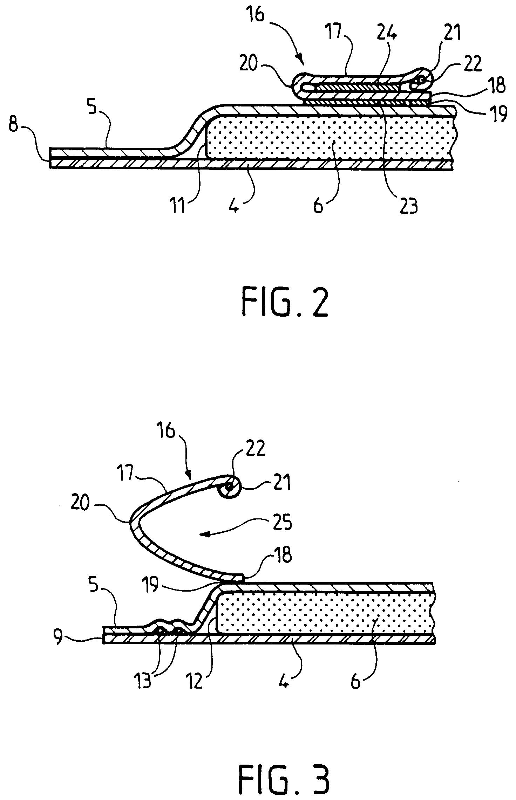

La structure générale de la couche-culotte suivant les figures 1 à 3 correspond à celle des couches-culottes classiques, comprenant, de l'extérieur vers l'intérieur, c'est-à-dire de bas en haut sur les figures 2 et 3, une feuille de support extérieure 4 imperméable aux liquides, une feuille de couverture intérieure 5 perméable aux liquides, et un coussin absorbant 6 disposé entre les deux feuilles 4 et 5.

Selon la figure 1, les deux feuilles 4 et 5 ont la même taille et la même forme, à savoir une forme générale rectangulaire avec deux bords transversaux 7 rectilignes opposés et deux bords longitudinaux 8 opposés comportant chacun une échancrure 9 sensiblement au milieu de sa longueur, ce qui donne aux deux feuilles 4 et 5 leur forme en sablier, les échancrures 9 définissant la partie d'entrejambes 3 de largeur réduite.

Le matelas ou coussin absorbant 6 disposé entre les deux feuilles 4 et 5, qui est également en forme de sablier, présente une taille réduite par rapport à celle des feuilles 4 et 5 et est centré par rapport à ces feuilles 4 et 5 de manière que ses bords transversaux 10 rectilignes ainsi que ses deux bords longitudinaux 11 chacun muni d'une échancrure 12 sensiblement au milieu de sa longueur, se trouvent respectivement en retrait vers l'intérieur par rapport aux bords correspondants 7 et 8 des deux feuilles 4 et 5.

La couche-culotte comporte, par ailleurs, de façon connue en soi, deux éléments élastiques 13 longitudinaux constitués, dans l'exemple représenté, chacun de deux brins élastiques espacés, fixés à l'état tendu à la feuille extérieure 4, au moins dans la partie d'entrejambes 3, entre le fond des échancrures 12 du coussin absorbant 6 et le fond des échancrures 9 des feuilles 4 et 5.

Deux attaches adhésives 14 sont prévues de façon connue en soi sur la partie arrière 1, en vue de la fermeture de la couche-culotte autour de la taille d'un utilisateur, les attaches 14 venant alors coopérer avec la partie avant 2 de la couche-culotte.

Les moyens de fixation du coussin absorbant 6 à la feuille extérieure 4 et de fixation de la feuille intérieure 5 à la feuille extérieure 4, autour du coussin 6, sont représentés sous la forme de lignes de colle 15 longitudinales. Par contre, on n'a pas représenté les moyens de fixation des éléments élastiques 13 à la feuille extérieure 4, ces moyens connus en soi pouvant être constitués par exemple par des lignes ou des enductions de colle.

On a par ailleurs représenté, sur la figure 1, l'axe médian longitudinal X-X de la couche-culotte.

La feuille intérieure 5 porte sur sa face intérieure, c'est-à-dire sa face visible sur la figure 1 qui est la face tournée vers l'utilisateur, deux volets ou rabats latéraux 16 formant barrière latérale, ces volets 16 étant espacés transversalement et disposés symétriquement de part et d'autre de l'axe longitudinal X-X.

Tel que cela apparaît surtout sur les figures 2 et 3, chaque volet 16 est formé d'un ruban 17 s'étendant sur toute la longueur de la couche-culotte, fixé sur toute sa longueur par son bord proximal 18, à la feuille intérieure 5 au-dessus du coussin 6, par collage, soudage ou tout autre procédé approprié, le long d'une ligne ou bande longitudinale 19, qui, dans la partie d'entrejambes 3, est située à l'intérieur du bord longitudinal du coussin 6, lequel bord est défini ici par le fond de l'échancrure 12.

A partir de la ligne de fixation 19, le ruban 17 s'étend vers l'extérieur et est replié symétriquement sur lui-même vers l'intérieur, sur toute sa longueur, suivant la ligne de pliage 20, de manière à former deux plis sensiblement égaux et que le bord distal 21 du ruban 17 se trouve sensiblement au-dessus du bord proximal 18. Le bord distal 21 du ruban 17 est élastifié au moins sur la partie médiane de la longueur du ruban, correspondant à la partie d'entrejambes 3. Dans l'exemple représenté, l'élément élastique 22 est fixé par collage dans une gaine ou un gousset formé par le repli sur lui-même du bord proximal du ruban.

Dans les deux parties d'extrémités 1 et 2 de la couche-culotte, le ruban 17 est fixé dans cette position repliée sur la feuille intérieure 5. Comme le montre la figure 2, cette fixation s'effectue par collage, soudage ou tout autre procédé approprié le long d'une première ligne transversale 23 par laquelle le pli inférieur du ruban 17 est rendu solidaire de la feuille intérieure 5, et le long d'une seconde bande transversale 24 par laquelle le pli supérieur du ruban 17 est rendu solidaire du pli inférieur de ce dernier.

Cette fixation du volet 16 en position repliée sur la feuille intérieure 5 de la couche-culotte, dans les deux parties d'extrémités 1 et 2 de cette dernière, assure que le volet 16, sous la tension de l'élément élastique 22, s'ouvre de la manière visible sur la figure 3 dans la partie d'entrejambes en constituant ici une poche 25 dont le bord libre élastifié défini par le bord distal 21 du ruban 17 se trouve positionné au-dessus du bord proximal 18 du volet 16. Par conséquent, les bords distaux 21 des deux volets opposés 16 se trouvent espacés l'un de l'autre d'une distance correspondant sensiblement à la distance séparant les bords proximaux 18 des deux volets 16 opposés.

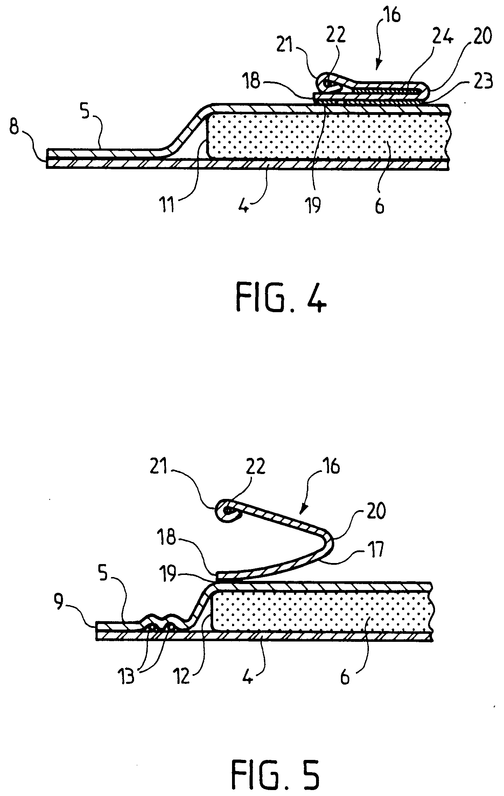

Dans le mode de réalisation suivant les figures 4 et 5, on retrouve la même structure générale de la couche-culotte selon les figures 1 à 3, avec une feuille de support extérieure 4 et une feuille de couverture intérieure 5 toutes deux en forme de sablier, un coussin absorbant 6 également en forme de sablier, des éléments élastiques longitudinaux 13 situés à l'extérieur du coussin 6 dans la partie d'entrejambes 3 de la couche-culotte, et deux volets latéraux 16 formés chacun d'un ruban 17 longitudinal. Ce ruban 17 est ici également relié à son bord proximal 18 par une bande ou ligne longitudinale 19 à la feuille intérieure 5, au-dessus du coussin absorbant 6, même dans la partie d'entrejambes de la couche-culotte, dans laquelle le coussin 6 comporte des échancrures latérales 12. De même, le bord distal 21 du ruban 17 est élastifié grâce à la présence d'un élément élastique 22 tendu et le ruban 17 est replié sur lui-même et fixé à la feuille intérieure 5, à l'état ainsi replié, dans les parties d'extrémités 1 et 2 de la couche-culotte par deux lignes ou bandes transversales 23 et 24.

Toutefois, dans ce mode de réalisation, le ruban 17 de chaque volet 16 est fixé par son bord proximal 18 à la feuille intérieure 5 de telle manière que le ruban 17 s'étende depuis ledit bord proximal vers l'intérieur de la couche-culotte, c'est-à-dire en direction du volet 16 opposé, et est replié autour de la ligne de pliage 20 vers l'extérieur, c'est-à-dire en sens inverse par rapport au mode de réalisation précédent. Ici également, le bord distal 21 élastifié de chaque volet 16 se trouve ainsi toujours positionné sensiblement au-dessus du bord proximal 18, même dans la partie d'entrejambes 3 de la couche-culotte dans laquelle le bord distal 21 du volet 16 peut se soulever de la feuille intérieure 5 sous l'action de son élément élastique 22.

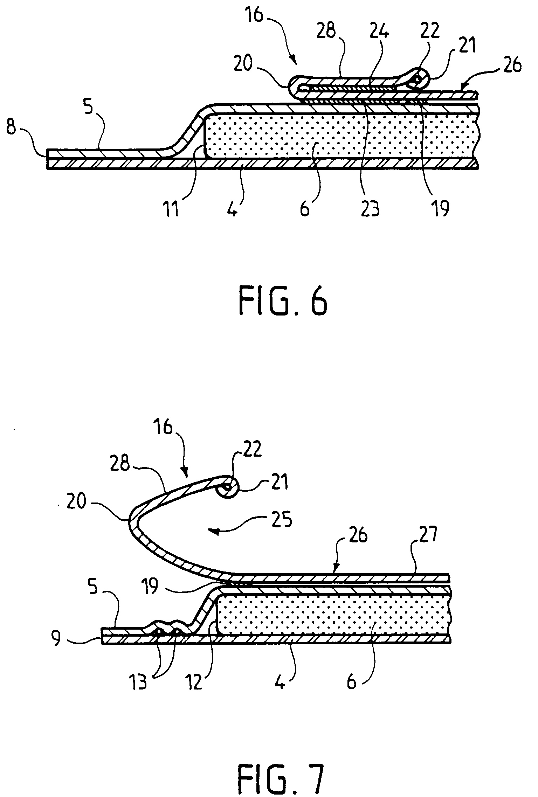

Dans la variante illustrée par les figures 6 et 7, on retrouve encore la structure générale de la couche-culotte suivant les figures 1 à 3, y compris les deux volets latéraux 16 qui s'étendent vers l'extérieur depuis leurs bords proximaux, définis par les lignes ou bandes longitudinales de fixation 19, et sont repliés sur eux-même vers l'intérieur. Par contre, les deux volets 16, au lieu d'être constitués chacun d'un ruban séparé, font ici partie d'un ruban longitudinal 26 unique dont la largeur est supérieure à la largeur du coussin absorbant 6 dans la partie d'entrejambes, c'est-à-dire à l'endroit des échancrures 12.

Ce ruban 26 est fixé le long des deux lignes longitudinales 19 sur la feuille intérieure 5, au-dessus du coussin absorbant 6, de manière que la partie médiane 27 du ruban 26, comprise entre les deux lignes 19, recouvre la feuille intérieure 5 au-dessus du coussin absorbant 6, et que les deux parties latérales 28 du ruban 26, situées au-delà des lignes 19, s'entendent vers l'extérieur. Chaque partie latérale 28 est repliée sur elle-même vers l'intérieur (ligne de pliage 20) et est élastifié à son bord distal 21, au moins dans la partie d'entrejambes de la couche-culotte, grâce à la présence d'un élément élastique 22 fixé à l'état tendu audit bord.

Ici également, les deux plis de chaque volet 16 sont, dans les deux parties d'extrémité de la couche-culotte, fixés à plat l'un sur l'autre par une ligne ou bande transversale 24 et sur la feuille intérieure 5 par une ligne du bande transversale 23, comme représenté sur la figure 6, de sorte que le bord distal 21 élastifié de chaque volet 16 puisse, dans la partie d'entrejambes de la couche-culotte, se soulever de la feuille intérieure 5 tout en restant positionné sensiblement au-dessus du bord proximal défini par la ligne 19. De cette manière, chacun des deux volets 16 s'ouvre sous la forme d'une poche 25 élastifiée sur son bord libre.

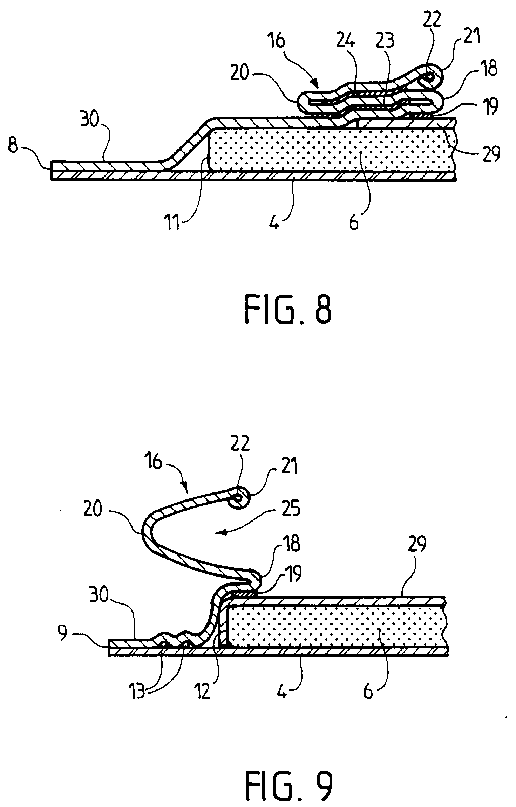

Selon le mode de réalisation des figures 8 et 9, la feuille Intérieure 5 qui, dans les modes de réalisation précédents, s'étend sur toute la largeur de la couche-culotte et recouvre donc le coussin absorbant 6 dans son ensemble, est remplacée par un ruban 29 d'une largeur constante légèrement supérieure à la largeur que le coussin 6 présente dans la partie d'entrejambes 3. Ce ruban 29 qui s'étend sur toute la longueur de la couche-culotte et est disposé en position médiane entre les bords longidinaux 8 opposés de la couche-culotte recouvre donc le coussin 6 sur toute la largeur dans la partie d'entrejambes 3, comme représenté sur la figure 9, et uniquement sur la partie médiane de la largeur dans la partie arrière 1 (et dans la partie avant 2), comme représenté sur la figure 8.

Chacun des deux volets 16 est ici réalisé d'une seule pièce avec un ruban latéral 30 qui, sur chaque côté de la couche-culotte, recouvre la feuille extérieure 4 et le coussin 6 sur leurs parties non recouvertes par le ruban médian 29 et remplace donc ici la feuille intérieure 5 des modes de réalisation précédents. Le ruban 30 est fixé selon la ligne ou bande longitudinale 19 au ruban 29, le long du bord latéral de ce dernier, et est prolongé latéralement au-delà de cette ligne 19 pour constituer le volet 16. Selon les figures 8 et 9, le ruban 30 est alors replié vers l'extérieur le long d'une première ligne de pliage, définissant le bord proximal 18 du volet 16, puis replié sur lui-même vers l'intérieur selon la ligne de pliage 20, le bord libre du ruban 30 étant replié sur un élément élastique 22 fixé au ruban 30 pour constituer le bord distal 21 élastifié du volet 16. Enfin, comme le montre la figure 8, le volet 16 est fixé à plat à l'état replié dans la partie arrière 1 (et dans la partie avant 2) de la couche-culotte par des lignes ou bandes transversales 23 et 24, alors que selon la figure 9, il est libre et peut donc se soulever pour former une poche 25 dans la partie d'entrejambes 3, son bord distal 21 restant néanmoins positionné sensiblement au-dessus de son bord proximal 18.

Bien entendu, dans ce mode de réalisation également, les volets 16, au lieu d'être repliés de manière à constituer des poches 25 ouvertes vers l'intérieur, pourraient être repliés de la manière illustrée par les figures 4 et 5.

Dans les modes de réalisation suivant les figures 1 à 3 et 4 et 5, les rubans 17 formant les volets 16 peuvent être réalisés à partir de feuilles de matériaux perméables aux liquides, par exemple des non tissés hydrophobes, ou de préférence imperméables aux liquides, par exemple des non tissés doublés d'un film ou d'une enduction imperméable.

Dans le mode de réalisation suivant les figures 6 et 7, le ruban 26 doit être constitué par un matériau perméable aux liquides qui peut être hydrophobe ou imperméable aux liquides dans la zone des parties latérales 28 constituant les volets 16. A titre d'exemple, le ruban 26 peut être constitué par un non-tissé hydrophobe rendu hydrophile uniquement dans la partie médiane 27. Ainsi, les deux couches de non-tissé (ruban 26 et feuille intérieure 5) recouvrant le coussin absorbant 6 dans la zone qui reçoit en premier les mictions, tout en facilitant la pénétration des fluides en direction du coussin absorbant, limitent les remontées de fluides, dans l'autre sens, Les parties latérales 28 constituant les volets 16, du fait de leur caractère hydrophobe, réduisent alors le remouillage, donc améliorent la "garde au sec". Dans l'exemple des figures 8 et 9, le ruban médian 29 perméable aux liquides peut être constitué, par exemple, par un non tissé rendu hydrophile, et les deux rubans latéraux 30 peuvent être constitués, par exemple, par un non tissé hydrophobe ou un non tissé rendu imperméable aux liquides, par exemple doublé par un film ou une enduction imperméable sur la face qui n'est pas en contact avec la peau de l'utilisateur, c'est-à-dire la face tournée vers la feuille extérieure de support 4.

Par ailleurs, il y a lieu de noter que pour constituer une feuille hydrophile dans sa partie médiane et hydrophobe dans ses deux parties latérales, comme c'est avantageusement le cas pour la feuille intérieure 5 des modes de réalisation des figures 1 à 3, 4 et 5 ainsi que 6 et 7 et pour le ruban 26 du mode de réalisation des figures 6 et 7, il est possible d'utiliser un non tissé ayant une bande ou zone longitudinale médiane présentant une structure différente de celle des deux bandes ou zones longitudinales latérales disposées de part et d'autre de cette bande médiane, cette structure différente étant obtenue pour une variation du rapport des deux composants (filaments et fibres) constituant le non tissé. Les propriétés hydrophiles et hydrophobes de ces bandes ou zones du non tissé peuvent également être obtenues par des traitements de surface par bandes ou zones du non tissé.

En outre, il faut remarquer que les lignes ou bandes de fixation 19, 23, 24, lorsqu'elles assurent une fixation par collage, peuvent être réalisées, soit par application au moment de la fixation de colle sous forme de colle en fusion (hot melt) sur les feuilles ou rubans à fixer, soit par réactivation, au moment de la fixation, de colle fusible thermo-réactivable appliquée préalablement à la fixation sur les feuilles ou rubans à fixer.

Enfin, si le bord distal 21 élastifié des volets 16 est défini, dans la description, comme étant situé sensiblement au-dessus du bord proximal 18 des volets, il y a lieu de noter que, dans le cadre de l'invention, les deux plis de chacun de ces volets peuvent avoir des largeurs inégales pouvant varier entre environ 1/3 et 2/3 de la largeur totale du volet entre le bord proximal et le bord distal.

Bien que l'invention ait été décrite ci-dessus et illustrée par les dessins annexés dans son application à une couche-culotte de forme anatomique ou en sablier comportant un coussin absorbant en forme de sablier, qui peut être destinée à des enfants ou des adultes incontinents, il va de soi qu'elle est applicable également à des couches-culottes qui ne sont pas en forme de sablier, ou qui comportent des coussins absorbants ayant une forme autre qu'en sablier, ainsi que d'une manière générale à tous les articles d'hygiène ayant la même structure générale que les couches-culottes.

## Revendications

1. Article d'hygiène absorbant jetable tel que couche-culotte, présentant une forme générale sensiblement rectangulaire avec des bords longitudinaux opposés (8) et des bords transversaux opposés (7), et comprenant, de l'extérieur vers l'intérieur, une feuille de support (4) imperméable aux liquides, un coussin absorbant (6) disposé et fixé sur la face intérieure de la feuille de support, les dimensions du coussin étant inférieures à celle de la feuille de support, une feuille de couverture (5) perméable aux liquides recouvrant au moins partiellement la face intérieure du coussin absorbant et fixée à la feuille de support au moins partiellement sur le pourtour du coussin absorbant, des éléments élastiques longitudinaux (13) fixés à l'état tendu à la feuille de support, à l'extérieur des bords longitudinaux (11) du coussin absorbant, deux volets latéraux (16) espacés transversalement disposés sur la face intérieure de la feuille de couverture, le long des bords longitudinaux (8) de l'article d'hygiène, lesdits volets (16) présentant chacun un bord proximal (18) relié à la feuille de couverture (5) et un bord distal (21) comportant des éléments élastiques longitudinaux tendus (22), chacun desdits volets (16) étant fixé à la feuille de couverture (5) le long de son bord proximal (18), sur toute la longueur de l'article d'hygiène, au voisinage de l'un des bords transversaux de l'article d'hygiène, pour fermer ce dernier autour de la taille d'un utilisateur de telle manière que l'article d'hygiène définisse une partie avant (2) et une partie arrière (1) correspondant respectivement aux deux zones d'extrémités proches desdits bords transversaux opposés (7) de l'article d'hygiène, et une partie d'entrejambes (3) correspondant à la zone intermédiaire située entre lesdites deux zones d'extrémités, caractérisé par le fait que : ledit bord proximal (18) se trouve au-dessus du coussin absorbant (6), sur toute la longueur de l'article d'hygiène, y compris dans la partie d'entrejambes et des moyens d'attache (14)
- chaque volet (16) est replié sur lui-même, sur toute sa longueur, suivant une ligne de pliage (20) située entre le bord proximal (18) et le bord distal (21) ;
- chaque volet (16) est fixé à plat dans cette position repliée à la feuille de couverture (5):
latéralement aux extrémités (1, 2) de l'article par des lignes ou bandes transversales (23, 24),
longitudinalement uniquement par son bord proximal (18), de manière que son bord distal (21) élastifié se trouve sensiblement au-dessus de son bord proximal (18), sur toute la longueur du volet, grâce à quoi le bord libre de chacun des volets (16) s'ouvre pour former une poche élastifiée (25).

2. Article d'hygiène suivant la revendication 1, caractérisé par le fait que chaque volet (16) est replié sur lui-même vers l'extérieur.

3. Article d'hygiène suivant la revendication 1, caractérisé par le fait que chaque volet (16) est replié sur lui-même vers l'intérieur.

4. Article d'hygiène suivant l'une quelconque des revendications précédentes, caractérisé par le fait que les deux volets (16) sont formés par deux rubans (17) séparés par un intervalle.

5. Article d'hygiène suivant l'une quelconque des revendications 1 à 3, caractérisé par le fait que les deux volets (16) sont formés par un seul ruban (26) dont les deux bords latéraux (21), définissant les bords distaux des volets, sont élastifiés et qui est fixé à la feuille de couverture (5) par deux lignes longitudinales (19) décalées vers l'intérieur par rapport auxdits bords latéraux et définissant les bords proximaux des volets, au moins une partie médiane (27) du ruban (26) situées entre lesdites lignes de fixation (19) étant perméable aux liquides.

6. Article d'hygiène suivant la revendication 5, caractérisé par le fait que ledit ruban (26) est constitué d'un voile perméable aux liquides, par exemple un non tissé, hydrophile dans ladite partie médiane (27) et hydrophobe ou imperméable aux liquides dans lesdites parties latérales (28).

7. Article d'hygiène suivant l'une quelconque des revendications 1 à 3, caractérisé par le fait que la feuille de couverture est constituée par un ruban (29) perméable aux liquides d'une largeur légèrement supérieure à la largeur du coussin (6) dans la partie d'entrejambes (3) recouvrant le coussin absorbant (6) et que chacun des deux volets (16) est constitué d'une seule pièce avec un ruban latéral (30) qui, sur chaque côté de la couche-culotte, recouvre les parties de la feuille de support (4) et du coussin (6) non recouvertes par le ruban médian (29), est fixé au ruban médian (29) par une ligne longitudinale (19) située au-dessus du coussin (6) définissant ledit bord proximal du volet et est prolongé au-delà de ladite ligne (19) sous la forme d'un volet (16) replié sur lui-même.

8. Article d'hygiène suivant la revendication 7, caractérisé par le fait que chaque ruban latéral (30) est constitué par un voile hydrophobe ou imperméable aux liquides.

## Claims

1. Disposable absorbent article of hygiene such as a nappy, exhibiting a substantially rectangular general shape with opposed lengthwise edges (8) and opposed transverse edges (7) and comprising, from the outside inwards, a liquid-impervious support sheet (4), an absorbent pad (6) arranged and fastened to the inner face of the support sheet, the dimensions of the pad being smaller than those of the support sheet, a liquid-permeable cover sheet (5) at least partially covering the inner face of the absorbent pad and fastened at least partially to the support sheet on the periphery of the absorbent pad, lengthwise elastic members (13) fastened in the stretched state to the support sheet, outside the lengthwise edges (11) of the absorbent pad, two transversely spaced side flaps (16) arranged on the inner face of the cover sheet, along the lengthwise edges (8) of the article of hygiene, each of the said flaps (16) having a proximal edge (18) connected to the cover sheet (5) and a distal edge (21) comprising stretched lengthwise elastic members (22), each of the said flaps (16) being fastened to the cover sheet (5) along its proximal edge (18), over the whole length of the article of hygiene, in the vicinity of one of the transverse edges of the article of hygiene, for closing the latter around a user's waist so that the article of hygiene defines a front part (2) and a rear part (1) corresponding respectively to the two end regions near the said opposed transverse edges (7) of the article of hygiene, and a crotch part (3) corresponding to the intermediate region situated between the two said end regions, characterized in that:
- the said proximal edge (18) is above the absorbent pad (6), over the whole length of the article of hygiene, including in the crotch part, and fastening means (14);
- each flap (16) is folded back onto itself, over its whole length, along a fold line (20) situated between the proximal edge (18) and the distal edge (21);
- each flap (16) is fastened flat in this folded-back position to the cover sheet (5):
laterally at the ends (1,2) of the article by transverse lines or strips (23,24),
lengthwise only by its proximal edge (18), so that its elasticized distal edge (21) is situated substantially above its proximal edge (18), over the whole length of the flap, by virtue of which the free edge of each of the flaps (16) opens to form an elasticized pouch (25).

2. Article of hygiene according to Claim 1, characterized in that each flap (16) is folded back onto itself outwards.

3. Article of hygiene according to Claim 1, characterized in that each flap (16) is folded back onto itself inwards.

4. Article of hygiene according to any one of the preceding claims, characterized in that the two flaps (16) consist of two tapes (17) separated by an interval.

5. Article of hygiene according to any one of Claims 1 to 3, characterized in that the two flaps (16) consist of a single tape (26) whose two side edges (21), defining the distal edges of the flaps, are elasticized and which is fastened to the cover sheet (5) by two lengthwise lines (19) set back inwards in relation to said side edges and defining the proximal edges of the flaps, at least a middle part (27) of the tape (26), which are situated between the said fastening lines (19) being permeable to liquids.

6. Article of hygiene according to Claim 5, characterized in that said tape (26) consists of a liquid-permeable tissue, for example a nonwoven, which is hydrophilic in said middle part (27) and hydrophobic or impervious to liquids in said side parts (28).

7. Article of hygiene according to any one of Claims 1 to 3, characterized in that the cover sheet consists of a liquid-permeable tape (29) of a width slightly greater than the width of the pad (6) in the crotch part (3) covering the absorbent pad (6), and in that each of the two flaps (16) consists of a single piece with a side tape (30) which, on each side of the nappy, covers the parts of the support sheet (4) and of the pad (6) which are not covered by the middle tape (29), is fastened to the middle tape (29) by a lengthwise line (19) situated above the pad (6) defining the said proximal edge of the flap and is extended beyond said line (19) in the form of a flap (16) folded back onto itself.

8. Article of hygiene according to Claim 7, characterized in that each side tape (30) consists of a hydrophobic or liquid-impervious tissue.

## Patentansprüche

1. Absorbierbarer Wegwerf-Hygieneartikel, beispielsweise Windelhose, mit einer im allgemeinen rechteckigen Form und gegenüberliegenden Längsrändern (8) und gegenüberliegenden Querrändern (7) und von außen nach innen betrachtet enthaltend: einen Halteboden (4), der für Flüssigkeiten undurchlässig ist, ein Absorptionskissen (6), das auf der Innenfläche des Haltebodens angeordnet und fixiert ist, wobei die Abmessungen des Kissens kleiner sind als diejenigen des Haltebodens, einen Abdeckboden (5), der für Flüssigkeiten durchlässig ist, zum zumindest teilweisen Abdecken der Innenfläche des Absorptionskissens, zumindest teilweise am Umfang des Absorptionskissens fixiert, elastische längliche Elemente (13), die im gespannten Zustand an dem Haltebogen fixiert sind, an der Außenseite der Längsränder (11) des Absorptionskissens, zwei seitliche Verschlußklappen (16), die quer beabstandet sind und auf der Innenfläche des Haltebodens entlang den Längsrändern (8) des Hygieneartikels angeordnet sind, wobei die Verschlußklappen (16) jeweils einen naheliegenden Rand (18) aufweisen, der mit dem Deckboden (5) verbunden ist, und einen fernliegenden Rand (21), der gespannte längliche elastische Elemente (23) enthält, und jede der Verschlußklappen (16) an dem Abdeckboden (5) entlang von dessen naheliegenden Rand (18) über die gesamte Länge des Hygieneartikels fixiert ist, in der Nähe eines der Querränder des Hygieneartikels, zum Schließen des letzteren um die Taille des Anwenders derart, daß der Hygieneartikel einen Vorderabschnitt (2) und einen Hinterabschnitt (1) definiert, jeweils gemäß den beiden Randzonen, die benachbart zu den gegenüberliegenden Querrändern (7) des Hygieneartikels vorliegen, sowie einen Zwischenbeinabschnitt (3) gemäß der Zwischenzone, die zwischen den beiden Randzonen vorliegt,
**gekennzeichnet** durch die Tatsache, daß:
der naheliegende Rand (18) oberhalb des Absorptionskissens (6) über die gesamte Länge des Hygieneartikels einschließlich dem Zwischenbeinabschnitt und der Befestigungseinrichtung (14) angeordnet ist,
jede Verschlußklappe (16) auf sich selbst über ihre gesamte Länge geklappt ist, entlang einer Faltlinie (20), die zwischen dem naheliegenden Rand (18) und dem fernliegenden Rand (21) angeordnet ist;
jede Verschlußklappe (16) glatt an dieser auf dem Abdeckbogen (5) umgeschlagenen Stelle fixiert ist:
quer an den Rändern (1, 2) des Artikels entlang der Querlienien oder -bänder (23, 24),
in Längsrichtung in eindeutiger Weise über den naheliegenden Rand (18) derart, daß sich der elastische fernliegende Rand (21) im wesentlichen oberhalb von dem naheliegenden Rand (18) befindet, über die gesamte Länge der Verschlußklappe hinweg, so daß sich hierdurch der freie Rand jeder Verschlußklappe (16) zum Bilden einer elastischen Tasche (25) öffnet.

2. Hygieneartikel nach Anspruch 1, dadurch gekennzeichnet, daß jede Verschlußklappe (16) auf sich selbst nach außen hin umgeklappt ist.

3. Hygieneartikel nach Anspruch 1, dadurch gekennzeichnet, daß jede Verschlußklappe (16) auf sich selbst nach innen hin umgeklappt ist.

4. Hygieneartikel nach einem der vorhergehenden Ansprüche, dadurch gekennzeichnet, daß die beiden Verschlußklappen (16) durch zwei Bänder (17) gebildet sind, die durch einen Abstand getrennt sind.

5. Hygieneartikel nach einem der vorhergehenden Ansprüche 1 bis 3, dadurch gekennzeichnet, daß die beiden Verschlußklappen (16) durch ein einziges Band (26) gebildet sind, von dem die beiden seitlichen Ränder (21), die die beabstandeten Ränder der Verschlußklappen bilden, elastisch ausgebildet sind, und das an dem Abdeckboden (5) über zwei Längslinien (19) fixiert ist, die nach innen hin im Hinblick auf die beiden Seitenränder stufenweise abgesenkt sind und die benachbarten Ränder der Verschlußklappen zumindest in einem Mittenabschnitt (27) des Bands (26) festlegen, der zwischen den beiden Fixierlinien (19) vorliegt und für Flüssigkeiten durchlässig ist.

6. Hygieneartikel nach Anspruch 5, dadurch gekennzeichnet, daß das Band (26) aus einem für Flüssigkeiten durchlässigen Flor gebildet ist, beispielsweise aus einem Vliesstoff, der in dem Mittenabschnitt (27) hydrophil ist und in den Seitenabschnitten (28) hydrophob oder für Flüssigkeiten undurchlässig ist.

7. Hygieneartikel nach einem der Ansprüche 1 bis 3, dadurch gekennzeichnet, daß der Abdeckboden durch ein Band (27) gebildet ist, das für Flüssigkeiten durchlässig ist und das Absorptionskissen (6) abdeckt, mit einer Größe, die im Zwischenbeinabschnitt (3) etwas größer als das Kissen (6) ist, und daß jede der beiden Verschlußklappen (16) in einem Stück mit einem Seitenband (30) gebildet ist, das auf jeder Seite der Windelhose die Teile des Haltebodens (4) und des Kissens (6) abdeckt, die nicht durch das Mittenband (29) abgedeckt sind, und an dem Mittenband (29) über eine Längslinie (19) fixiert ist, die oberhalb des Kissens (6) vorliegt, unter Definierung des naheliegenden Rands der Verschlußklappe, und das über die Linie (19) in Form einer auf sich selbst geklappten Verschlußklappe (16) verlängert ist.

8. Hygieneartikel nach Anspruch 7, dadurch gekennzeichnet, daß jedes Seitenband (30) durch einen hydrophoben oder für Flüssigkeiten undurchlässigen Flor gebildet ist.
